# EUROPEAN PATENT APPLICATION

(11) **EP 2 033 573 A2**
(43) Date of publication of application: **11.03.2009**
(21) Application number: 08163942.9
(22) Date of filing: 09.09.2008
(51) Int. Cl.: A61B 5/00

(54) **Performance measuring system, data transmission method and computer program**

(30) Priority: 10.09.2007 FI 20075629
(71) Applicant: Polar Electro Oy, 90440 Kempele (FI)
(72) Inventor: Rytky, Pekka, 90240, Oulu (FI)
(74) Representative: Karppinen, Olavi Arto

(57) **Abstract**

A portable user-specific performance measuring system (100) comprises a quality unit (110) which receives a measurement signal (120) and measures the quality of the measurement signal (120), and a presentation unit (136) which receives from the quality unit (110) quality data (140) indicating the quality of the measurement signal (120) and presents the quality of the measurement signal (120) on the basis of the quality data (140).

## Description

### FIELD

The invention relates to a portable, user-specific performance measuring system, a related method for data transmission of signal quality and a corresponding computer program.

### BACKGROUND

It is important to measure the functioning of the organ system and the movement of a person when training for an athletic performance, for example. The heart rate of a person, for instance, can be measured with a portable device that comprises a measuring belt to be fastened on the chest with an elastic belt and a heart rate receiver to be fastened on the wrist like a watch, which heart rate receiver serves as a receiver of measurement data and may process the measurement data.

The measuring belt consists of a flexible piece to be fastened on the chest and having a sensor for measuring an ECG signal. The sensor comprises two measuring electrodes that settle against the skin and are connected to an ECG detecting block. Instead of ECG measurement or in addition to it, it is possible to use several other sensors to measure the state of the organ system or movement of the user. These include acceleration sensors that provide information on the movements of the user.

A transmitter in the measuring belt or some other sensor may wirelessly transmit measurement data to the measurement data receiver fastened to the wrist of the user for processing therein. From the measuring belt or wrist receiver, the measurement data may be transmitted on to a computer or some other corresponding receiver for further processing.

However, there are problems associated with the measurement. Sweating during an athletic performance moistens the skin and improves its electrical conductivity, whereas at the beginning of the athletic performance the skin may be dry and have poor conductivity. In the latter case, the contact of the measuring electrodes of the measuring belt with the skin may be weak, which deteriorates the quality of the measurement signal received from the electrodes. There are also several other reasons affecting the contact between the skin and the electrodes, such as the tightness of the measuring belt, due to which the user cannot know how good or bad the contact is. It is also possible that a proper measurement signal is not received at all due to the poor contact, the measurement signal being only noise. Since the measuring result depends on the contact and the consequent quality of the measurement signal, measuring the real functioning of the user's organ system may be insecure or fail altogether, particularly at the beginning of an athletic performance.

### BRIEF DESCRIPTION

An object of the invention is to implement a method for measurement of signal quality for a performance measuring system. This is achieved by a data transmission method during the performance of which a sensor of a portable user-specific performance measuring system is intended to be in contact with a user to generate a measurement signal relating to the functioning of the user's organ system. The method comprises receiving quality data of the measurement signal generated by the sensor; and presenting the quality of the measurement signal to the user on the basis of the quality data.

A further object of the invention is a portable user-specific performance measuring system arranged to generate a measurement signal relating to the functioning of a user's organ system when the performance measuring system is being used by the user. The performance measuring system comprises: a presentation unit arranged to receive quality data indicating the quality of the measurement signal and to present the quality of the measurement signal on the basis of the quality data.

Yet another object of the invention is a computer software product comprising encoded instructions which, loaded on a portable user-specific performance measuring system, form a computer process controlling the operation of the performance measuring system, the performance measuring system being intended to measure the functioning of a user's organ system. The computer process comprises receiving the quality data of a measurement signal generated by a sensor; and presenting the quality of the measurement signal to the user on the basis of the quality data.

Preferred embodiments of the invention are described in the dependent claim.

Several advantages are achieved with the method and performance measuring system according to the invention. The quality of the measurement signal can be presented to the user, and the user can, on the basis of these data, take measures to improve the contact and the quality of the measurement signal, if required.

### LIST OF FIGURES

The invention will now be described in greater detail in connection with preferred embodiments, referring to the attached drawings, in which
Figure 1 shows a measuring unit;
Figure 2 shows a measuring unit provided with a processor;
Figure 3 shows a measuring unit in which a processor modifies both the measurement signal and the quality data;
Figure 4 shows a measuring unit provided with a presentation unit;
Figure 5 shows a quality unit controlling a presentation unit;
Figure 6 shows a performance measuring system comprising a measuring unit and a central processing unit;
Figure 7 shows a user using a performance measuring system;
Figure 8 shows a central processing unit showing that the quality of the measurement signal is sufficiently good;
Figure 9 shows a central processing unit showing the quality of the measurement signal with a bar;
Figure 10 shows a measuring unit showing the quality of the measurement signal with two LEDs;
Figure 11 shows a measuring unit showing the quality of the measurement signal with a row of LEDs; and
Figure 12 shows a flow chart of the method.

### DESCRIPTION OF EMBODIMENTS

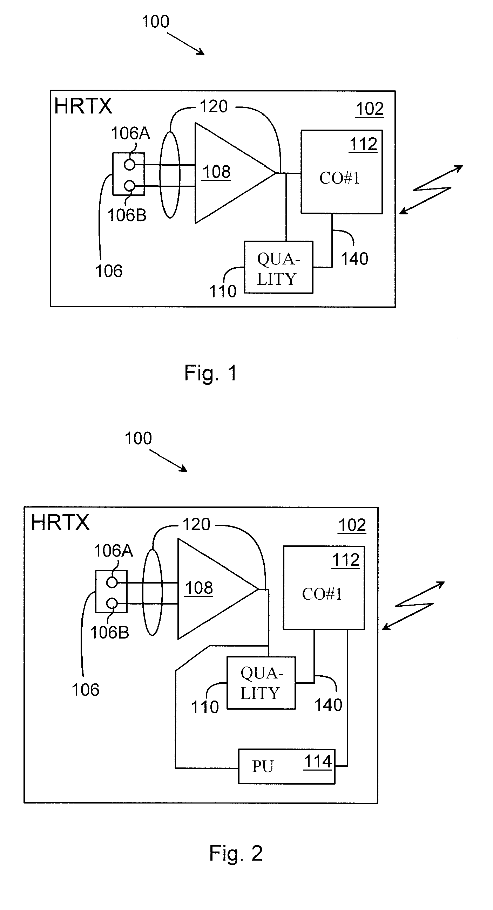

First, let us examine measurement of the signal quality with reference to Figures 1 to 3. The performance measuring system 100 may comprise a measuring unit 102, which may comprise one or more sensors 106 measuring the functioning of one or more organ systems, a preamplifier 108, a quality unit 110 and a communication unit (CO#1) 112.

The sensor 106 may measure the functioning of the organ system. The functioning of the organ system may be represented by the heart rate etc.

When measuring the heart rate, for example, electrodes 106A, 106B of the sensor 106 may detect the voltage generated by the electrical activity of the heart muscle from the user's skin surface and generate a measurement signal, such as an ECG signal (ECG, electrocardiogram) representing the electrical activity of the heart muscle.

The measurement signal may be fed from the electrodes 106A, 106B to the ECG preamplifier 108, which can amplify the measurement signal 120 generated by the sensor 106 and feed the amplified measurement signal to the communication unit 112 and quality unit 110. The preamplifier 108 may comprise several amplification stages. ECG data, for example, may be processed in such a way that they comprise the ECG as such, a part of the ECG and/or timing data of the heart rate. The timing data may contain a timing pulse representing a predetermined part of the ECG.

To provide quality data, the quality unit 110 may measure the amplitude of the measurement signal 120. As a quality-representing property, the quality unit 110 may alternatively or additionally measure from the measurement signal the signal-to-interference ratio or the shape of the measurement signal 120 in relation to a predetermined reference shape. Here, high amplitude is good, meaning a voltage of more than 1 mV from peak to peak between the electrodes 106A, 106B. A good signal-to-interference ratio also means good quality. In this case, the low-frequency (e.g. below 10 Hz) interference has been filtered out and the measurement may be carried out in the frequency range of 10 Hz to 30 Hz. Also, the closer to the predetermined reference shape the measurement signal shape is, the better the quality of the measurement signal can be considered. The reference signal may be generated from the user's own measurement signal before the actual performance measurement. To generate the reference signal, the measurement may be carried out under predetermined conditions, such as in rest. The reference signal may be measured only once, repeatedly from time to time or every time before the actual performance measurement. The comparison may be visual or done by means of a computer program. The measurement signal shape and the predetermined reference shape can be compared by means of correlation, for example.

The communication unit 112 may comprise several successive amplification stages, such as an AGC (Automatic Gain Control) amplifier and a power amplifier. The communication unit 112 transmits as wireless data transmission a signal comprising measurement data and quality data. The measuring unit 102 may also be a transceiver, in which case the communication unit 112 may wirelessly receive signals sent by a predetermined communication party. Wireless communication may be based on magnetic pulses or radio-frequency electromagnetic radiation. The frequency of radio-frequency data transmission may be 2.4 GHz, and the data transmission may be digital data transmission, the data packet of which may have a separate field for quality data.

Figure 2 shows an embodiment of the measuring unit 102 of the performance measuring system 100. The measuring unit 102 may also comprise a processing unit (PU) 114, which may convert a measurement signal 120 to be transferred to the communication unit 112 into a digital form and process it digitally in a desired manner. It may be filtered and/or encoded, for example.

The processing unit 114 may be implemented by using analogue ciruits, ASIC circuits (Application Specific Integrated Circuit), a digital processor, memory and computer software. The processing unit 114 may form a part of the computer of the performance measuring system 100.

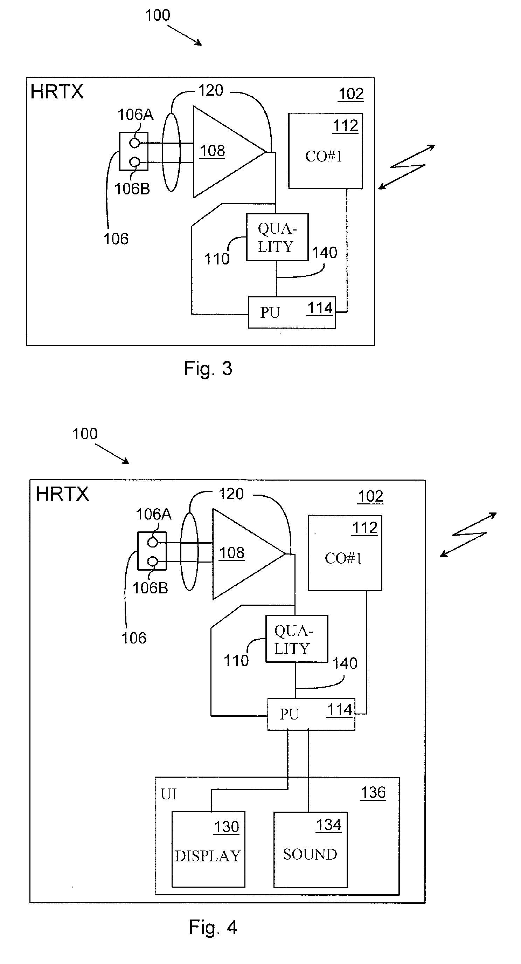

As presented in Figure 3, the measuring unit 102 may comprise a processing unit (PU) 114 that may convert quality data 140 provided by the quality unit 110 as well as measurement data, which are to be transferred to the communication unit 112, into a digital form and process these data as desired by means of digital signal processing. They may be filtered and/or encoded, for example.

Let us now examine, with reference to Figure 4, presentation of the quality of the measurement signal 120 in the measuring unit 102. The measuring unit 102 may comprise a presentation unit 136. The presentation unit 136 may be controlled, according to the quality encoded in the quality data 140, by means of a processing unit 114 that may be a processor, for instance. The presentation unit 136 may comprise a display 130 and/or a sound generator 134.

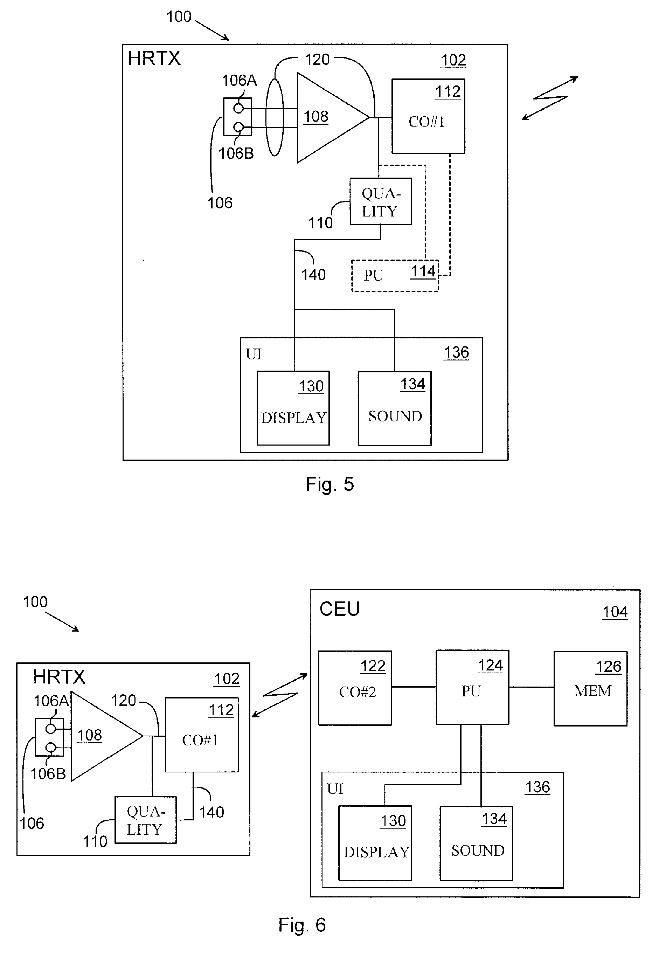

The display 130 may be, for example, an LCD display (Liquid Chrystal Display) or a row of LEDs. An LCD display may, controlled by the processing unit 114, display the quality of the measurement signal 120 graphically and/or numerically. The processing unit 114 may also control a row of LEDs serving as a presentation unit (see Figures 4 and 5).

The sound generator 134 may be, for example, a loudspeaker or a piezoelectric crystal generating, controlled by the processing unit 114, an audible audio signal to present the quality. The audio signal may be speech expressing the quality. Alternatively or additionally, the audio signal may be an audible call expressing quality.

Let us now examine presentation of the quality of the measurement signal 120 in the measuring unit 102 with reference to Figure 5. In this embodiment, the presentation unit 136 may be controlled according to the quality data of the quality unit 110 without a processing unit 114. A processing unit 114 is not necessarily needed in this embodiment but the measurement signal 120 may be fed directly to the communication unit 112, as in Figure 1.

Figure 6 shows the measuring unit 102 and the central processing unit 104, which may together form a user-specific performance measuring system 100. In this example, the measuring unit 102 is according to Figure 1 but it may also be according to Figures 2 to 4B. The central processing unit 104 may comprise a communication unit (CO#2) 122, a processing unit (PU) 124, a memory unit (MEM) 126 and a presentation unit 136. If the central processing unit 104 comprises a presentation unit 136, there is usually no presentation unit 136 in the measuring unit 102 but this does not have to be the case.

The communication unit 112 may comprise a coil to which the preamplifier 108 may feed quality data 140 generated by the quality unit 110 and measurement data received from the sensor 106, which measurement data correspond to, for example, the heart rate when the functioning of the heart is being measured. By means of the coil, a magnetic signal to be transmitted can be generated which contains measurement data and quality data. The coil thus forms a magnetic field varying in phase with the heart rate, the quality of the heart rate being encoded amid the magnetic field. The coil of the communication unit 112 may be in inductive communication with the coil of the communication unit 122, for example, and thus the measuring unit 102 may transfer both the measurement data and the quality data to the central unit 104.

The communication unit 112 of the measuring unit 102 may also transmit the measurement data and quality data to the communication unit 122 of the central processing unit 104 as electromagnetic radiation.

The communication unit 122 of the central processing unit 104 may receive the measurement data and quality data and feed these data to the processing unit 124, which may execute a computer process according to the encoded instructions stored in the memory unit 126.

The processing unit 124 may be implemented by using analogue circuits, ASIC circuits (Application Specific Integrated Circuit), a digital processor, memory and computer software. The processing unit 124 may comprise a part of the computer of the performance measuring system 100.

By means of its communication unit 112, the measuring unit 102 may transmit to the communication unit 122 of the central processing unit 104 not only the measurement data but also the quality data that indicate the quality of the measurement signal 120, measured by the quality unit 110 of the measuring unit 102. The communication unit 122 may feed the quality data 140 to the processing unit 124, which controls, similarly to the processing unit 114 in the case of Figure 4A, the presentation unit 136 to present the quality of the measurement signal 120. If no processing unit 124 is in use, it is possible that the presentation unit 136 is controlled by the communication unit 122 on the basis of the quality data 140.

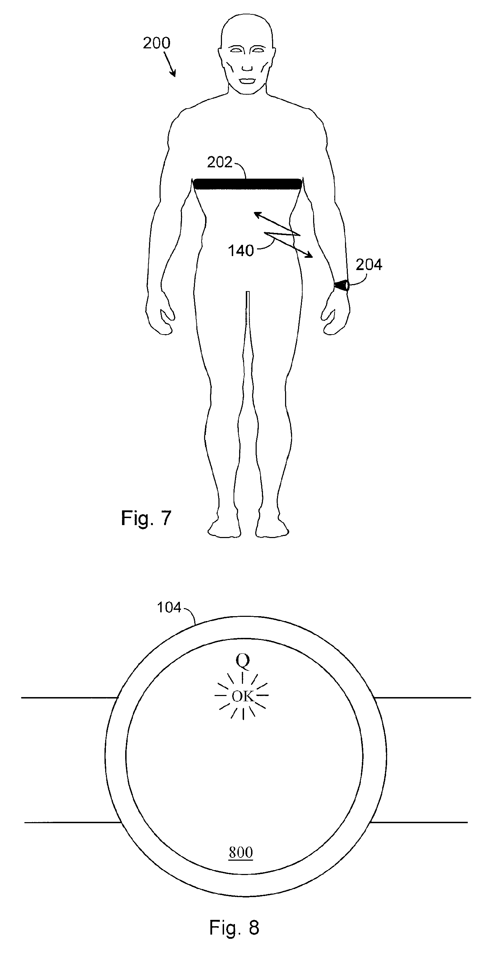

With reference to the embodiment shown in Figure 7, the measuring transmitter 102 may be a transmitter belt 202 fastenable around a user's 200 chest. By means of magnetic pulses or radio-frequency electromagnetic radiation, the quality data 140 may be transmitted from the transmitter belt 202 for example to a wrist-held device 204, which may be the same as the central unit 104. Instead of on the wrist, the device 204 may also be positioned elsewhere, for instance on a bicycle. The device 204 may receive signal corresponding to the ECG signal measured by the electrodes 106A, 106B and determine the quality of this signal. At its simplest, the performance measuring system may comprise only a wrist-held device 204 with electrodes. In such a case, the wrist-held device 204 corresponds to both the measuring unit 102 and the central processing unit 104, and it may measure the functioning of the organ system and, at the same time, express the quality of the measurement signal to the user.

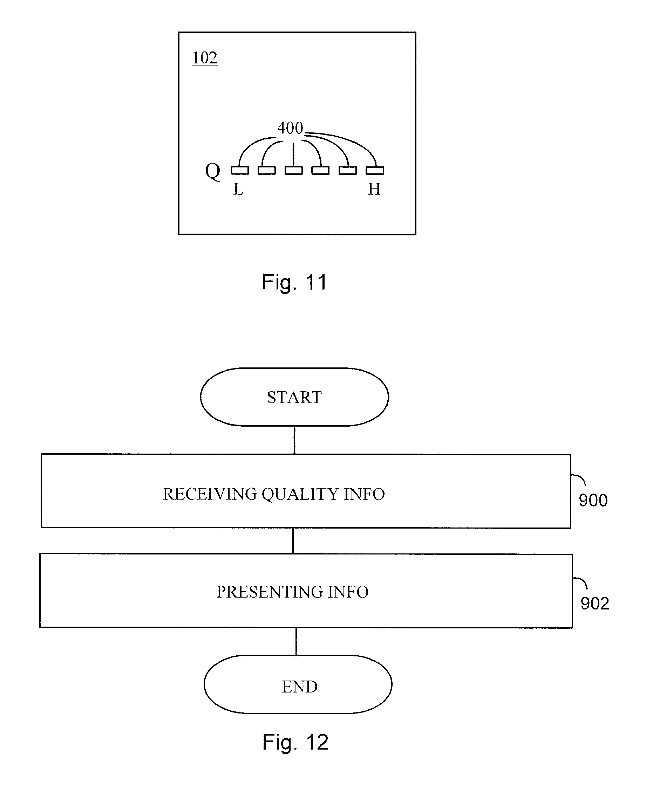

Figures 8 to 11 show some examples of presenting the quality of the measurement signal. Figure 11 shows a central processing unit 104 (or measuring unit 102), on the liquid crystal display 800 of which an "OK" sign or the like is activated when the quality of the measurement signal is good for reliable operation. An inactivated "OK" sign means hereby that the quality of the measurement signal is not sufficiently good for reliable operation. Activation may mean that the "OK" sign is (clearly) perceivable, whereas an inactivated "OK" sign is not (clearly) perceivable.

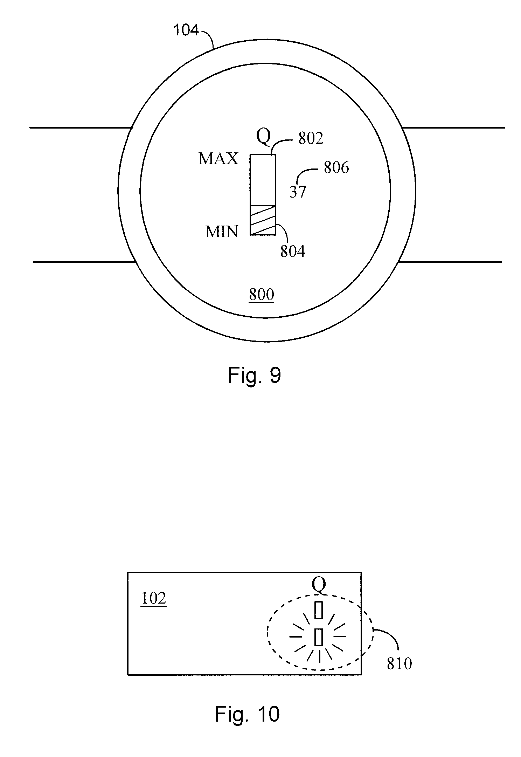

Figure 9 shows an example of a bar chart 802 on the liquid crystal display 800 of the central processing unit 104 (or measuring unit 102), where the height of the chart part 804 representing the quality of the measurement signal changes according to the quality of the measurement signal. If the quality of the measurement signal is excellent, the part 804 representing the quality may extend to the greatest possible height (MAX). If, on the other hand, the quality of the measurement signal is weaker, the part 804 representing the quality of the measurement signal may correspondingly be lower. Instead of a vertical bar, also a horizontal bar may represent the quality of the measurement signal. Instead of or in addition to a vertical or horizontal bar, numerals 806 may represent the quality.

Figure 10 shows a LED display 810 of the central processing unit 104 (or measuring unit 102), comprising two LEDs. Activating the lower LED may mean that the quality of the measurement signal is too poor for reliable measurement. The lower LED may be red, for example. Activating the upper LED may mean that the quality of the measurement signal is sufficiently good for reliable measurement. The upper LED may be green, for example.

Figure 11 shows an embodiment where the quality of the measurement signal is indicated by a row of LEDs 400. The better the quality of the measurement signal is, the more LEDs are activated. The activated LEDs radiate visible light, and the inactivated LEDs have been switched off. The scale implemented by means of LEDs may show a range of 0 to 3 mV. Each LED may correspond to 0.5 mV, for example, in which case the embodiment according to the example requires six LEDs.

Figure 12 shows a flow chart of the method. In step 900, the quality of the measurement signal is measured. In step 902, the quality of the measurement signal is presented to the user.

The method shown in Figure 12 can be implemented as a logical circuit solution or a computer program. A computer program may be positioned on a distribution medium of a computer program for distribution. A distribution medium of a computer program is readable by a data processing device, and it encodes the computer program commands for controlling the operation of the performance measuring system.

The distribution medium, in turn, may be a solution known as such for distributing a computer program, for example a medium readable by a data processing device, program-storing medium, memory readable by a data processing device, software distribution package readable by a data processing device, signal readable by a data processing device, data communication signal readable by a data processing device or compressed software package readable by a data processing device.

Once the quality of the measurement signal has been presented to the user, the user may correct the fastening, position and/or tightness of the measuring unit 102 if he/she wishes to improve the quality of the measurement signal. Additionally or alternatively, the user may also moisten the electrodes of the measuring unit and/or the skin to improve the contact and the quality of the measurement signal.

Although the invention has been described above with reference to the examples according to the attached drawings, it is clear that the invention is not restricted to them but may be modified in a plurality of ways within the scope of the attached claims.

## Claims

1. A data transmission method during the performance of which a sensor (106) of a portable user-specific performance measuring system (100) is intended to be in contact with a user (200) to generate a measurement signal relating to the functioning of the user's (200) organ system, **characterized by** the method comprising:
receiving (900) quality data (140) of the measurement signal (120) generated by the sensor (106); and
presenting (902) the quality of the measurement signal (120) to the user on the basis of the quality data (140).

2. A method according to claim 1, **characterized by** the performance measuring system (100) comprising a measuring unit (102) and a central processing unit (104) which are physically separate, and of which the measuring unit (102) comprises a sensor (106), a quality unit (110) and a communication unit (112), and of which the central processing unit (104) comprises a communication unit (112) and a presentation unit (130, 134); and
receiving a measurement signal (120) by a quality unit (110);
measuring the quality of the measurement signal (120) by the quality unit (110);
generating by the quality unit (110) the quality data (140) indicating the quality of the measurement signal (120);
receiving the quality data (140) by the communication unit (112), and transmitting the quality data (140) of the measurement signal from the communication unit (112) of the measuring unit (102) to the communication unit (122) of the central processing unit (104) to present the quality on a presentation unit (136).

3. A method according to claim 1, **characterized by** measuring by a quality unit (114) the quality of the measurement signal (120) generated by the sensor (106), and generating by the quality unit (114) quality data (140) characterizing the quality of the measurement signal (120).

4. A method according to claim 1, **characterized by** measuring the amplitude of the measurement signal as a quality-representing property.

5. A method according to claim 4, **characterized by** presenting the quality by showing the amplitude of the measurement signal (120).

6. A method according to claim 1, **characterized by** measuring from the measurement signal (120) the signal-to-interference ratio as a quality-representing property.

7. A method according to claim 6, **characterized by** presenting the quality by showing the signal-to-interference ratio.

8. A method according to claim 1, **characterized by** measuring the shape of the measurement signal (120) to determine a quality-representing parameter.

9. A method according to claim 8, **characterized by** presenting said at least one property by showing the parameter determining the shape of the measurement signal (120).

10. A method according to claim 8, **characterized by** comparing the shape of the measurement signal (120) with the reference signal generated earlier from a measurement signal measured under predetermined conditions.

11. A method according to claim 1, **characterized by** presenting the quality by means of an audio signal.

12. A method according to claim 11, **characterized by** the audio signal being speech.

13. A method according to claim 11, **characterized by** the audio signal being an encoded audible call.

14. A portable user-specific performance measuring system arranged to generate a measurement signal (120) relating to the functioning of a user's organ system when the performance measuring system (100) is being used by the user (200), **characterized in that** the performance measuring system (100) comprises:
a presentation unit (136) arranged to receive quality data (140) indicating the quality of the measurement signal (120) and to present the quality of the measurement signal (120) on the basis of the quality data (140).

15. A performance measuring system according to claim 14, **characterized in that** the performance measuring system (100) comprises a measuring unit (102) and a central processing unit (104) which are physically separate, and of which the measuring unit (102) comprises a sensor (106), a quality unit (110) and a communication unit (112), and the central processing unit (104) comprises a communication unit (122) and a presentation unit (136); and
the communication unit (112) of the measuring unit (102) is arranged to receive the quality data (140) from the quality unit (110) and to transmit the quality data (140) to the central processing unit (122) to present the quality of the measurement signal (120) on the presentation unit (136).

16. A performance measuring system according to claim 14, **characterized in that** the performance measuring system comprises a quality unit (110) arranged to receive the measurement signal (120), to measure the quality of the measurement signal (120) and to generate quality data (140) characterizing the quality of the measurement signal (120).

17. A performance measuring system according to claim 14, **characterized in that** the quality unit (110) is arranged to measure the amplitude of the measurement signal as a quality-representing property.

18. A performance measuring system according to claim 14, **characterized in that** the quality unit (114) is arranged to measure from the measurement signal (120) the signal-to-interference ratio as a quality-representing property.

19. A performance measuring system according to claim 14, **characterized in that** the quality unit (110) is arranged to measure the shape of the measurement signal (120) to determine a quality-representing parameter.

20. A performance measuring system according to claim 17, 18 or 19, **characterized in that** the presentation unit (136) comprises a display (130) arranged to show the quality of the measurement signal (120).

21. A performance measuring system according to claim 19, **characterized in that** the quality unit (110) is arranged to compare the shape of the measurement signal (120) with a reference signal generated earlier from a measurement signal measured under predetermined conditions.

22. A performance measuring system according to claim 14, **characterized in that** the presentation unit (130, 134) comprises a sound generator (134) arranged to present an audio signal according to the quality of the measurement signal (120).

23. A performance measuring system according to claim 22, **characterized in that** the audio signal is speech expressing the quality.

24. A performance measuring system according to claim 22, **characterized in that** the audio signal is an audible call expressing the quality.

25. A computer software product comprising encoded instructions which, loaded on a portable user-specific performance measuring system (100), form a computer process controlling the operation of the performance measuring system (100), the performance measuring system (100) being intended to measure the functioning of a user's (200) organ system, **characterized in that** the computer process comprises
receiving the quality data of a measurement signal (120) generated by a sensor (106); and
presenting the quality of the measurement signal (120) to the user on the basis of the quality data.

26. A computer software product according to claim 25, **characterized in that** the performance measuring system (100) comprises a measuring unit (102) and a central processing unit (104) which are physically separate, and of which the measuring unit (102) comprises a sensor (106), a quality unit (114) and a communication unit (112), and of which the central processing unit (104) comprises a communication unit (112) and a presentation unit (136); and the computer process comprises
receiving the measurement signal (120) by a quality unit (110);
measuring the quality of the measurement signal (120) by the quality unit (110);
generating by the quality unit (110) quality data (140) indicating the quality of the measurement signal (120);
receiving the quality data (140) by the communication unit (112), and transmitting the quality data (140) from the communication unit (112) of the measuring unit (102) to the communication unit (122) of the central processing unit (104) to present the quality on the presentation unit (136).

27. A computer software product according to claim 25, **characterized in that** the quality of the measurement signal (120) generated by the sensor (106) is measured by the quality unit (114), and the quality data (140) characterizing the quality of the measurement signal (120) is generated by the quality unit (114).
